# EUROPEAN PATENT APPLICATION

(11) **EP 2 360 239 A2**
(43) Date of publication of application: **24.08.2011**
(21) Application number: 11001418.0
(22) Date of filing: 28.01.2005
(51) Int. Cl.: C12N 1/20, C12S 1/00, B09C 1/10, C02F 3/34, C12R 1/01, A62D 3/00, C12N 1/26

(54) **Surfactant biocatalyst for remediation of recalcitrant organics and heavy metals**

(62) Divisional of application: 05722704.3
(71) Applicant: Savannah River Nuclear Solutions, LLC, Aiken, SC 29808 (US)
(72) Inventor: Story, Sandra, Travelers Rest South Carolina 29690 (US); Brigmon, Robin L, North Augusta South Carokina 29841 (US); Altman, Denis, Evans Georgia 30809 (US); Berry, Christopher J, Aiken South Carolina 29803-5743 (US)
(74) Representative: Robertson, James Alexander

(57) **Abstract**

Novel strains of isolated and purified bacteria have been identified which have the ability to degrade petroleum hydrocarbons including a variety of PAHs. Several isolates also exhibit the ability to produce a biosurfactant. The combination of the biosurfactant-producing ability along with the ability to degrade PAHs enhances the efficiency with which PAHs may be degraded. Additionally, the biosurfactant also provides an additional ability to bind heavy metal ions for removal from a soil or aquatic environment.

## Description

### STATEMENT AS TO RIGHTS TO INVENTIONS MADE UNDER FEDERALLY SPONSORED RESEARCH AND DEVELOPMENT

This invention was made with Government support under Contract No. DE-AC09-96SR18500 awarded by the United States Department of Energy. The Government has certain rights in the Invention.

### FIELD OF THE INVENTION

This invention is directed towards bacterial strains useful for bioremediation and processes for using the bacterial strains. In particular, it relates to unique bacterial isolates that can degrade polyaromatic hydrocarbons (PAHs) and methods to use these novel bacterial strains for bioremediation. Remediation of lead by reducing bioavailability has also been demonstrated by one of the strains indicating dual use for organic and inorganic remediation.

### BACKGROUND OF THE INVENTION

Various scientific articles and patents are referred to throughout the specification. These publications are incorporated herein by reference to describe the state of the art to which this invention pertains and to provide details on standard methodologies and apparatuses which may be useful in practicing various embodiments of the present invention.

Polyaromatic hydrocarbons (PAHs) are widespread, common pollutants particularly found in association with oil refineries, certain refined petroleum products, petroleum storage locations, and petroleum spill sites. High levels of PAHs are associated with mutagenic and carcinogenic effects in humans and pose a high risk for migration to and pollution of soil and ground water sources. As a result, there has been considerable interest in techniques and processes which will degrade PAHs and related petroleum products to remediate the environment. The uses of biological agents to treat PAHs are well known within the art. US Pat. No. 6,503,746 to Daane describes bacterial strains in the family *Bacillaceae* which are used in PAH remediation efforts.

US Pat. No. 3,616,204 to Linn discloses inoculating contaminated soil with cultures of microorganisms known to degrade the unwanted contaminants. The procedure described in Linn additionally involves introducing nutritional supplements to increase the soil remediation efficiency.

US Pat. No. 5,100,455 to Pickard discloses using indigenous microflora and fauna in combination with humic substrates to biologically treat soil contaminants including petroleum associated hydrocarbons.

US Pat. No. 4,849,360 to Norris discloses a bioreactor for treating petroleum contaminated soil in which air is forced through the contaminated soil to facilitate the bioremediation. The bioreactor uses indigenous microflora which are supplemented with phosphorus and nitrogen nutrients.

While a variety of PAH-degrading bacteria are known and have been utilized in various applications for remediation, there remains a need for improvement in the art in terms of identifying new and useful species having novel properties which are effective for the rapid degradation of petroleum pollutants. Further, there remains a need for improvements directed to the field of metal remediation such as by reducing the bioavailability of metal present within contaminated soils, metal-containing waste streams, and industrial process materials.

### SUMMARY OF THE INVENTION

The present invention relates to methods for the degradation of petroleum pollutants including polyaromatic hydrocarbons (PAHs). Additionally, the present invention relates to a biotreatment process which enhances the removal of heavy metals from soil. The present invention uses isolated and purified bacterial strains of bacterial isolates from an oil refinery field. Certain of the isolates having a further ability to produce useful biosurfactants.

It is one aspect of at least one of the present embodiments of the present invention to provide isolated bacterial strains that produce biosurfactants under *in situ* and *ex situ* remediation conditions. The innate ability of the isolated and purified bacterial strains to produce biosurfactants contributes to the remediation properties of the bacteria. The biosurfactant provides increased solubility of PAHs and access of the bacteria to the PAHs, thereby increasing the efficiency of the bioremediation by the bacteria strains. The biosurfactant can also reduce soil Pb bioavailability and is believed to have utility in bioremediation of other metal contaminants which also respond to surfactant treatments.

An additional aspect of at least one of the embodiments of the present invention is related to isolated and purified strains of bacteria in which the surfactant producing properties contribute to enhanced solubilization of petroleum and petroleum-derived products. The biosurfactants increase the solubilization of the petroleum products which promotes the aqueous flushing or removal of petroleum products associated with biosurfactant aggregates such as micelles and related structures. Further, the biosurfactants also increase the bioavailability of petroleum products that enhance the microbial ability to degrade contaminants. The enhanced bioavailability is beneficial to the isolated and purified strains as well as other beneficial microorganisms present in the contaminated substrate.

It is yet another aspect of at least one of the embodiments of the present invention to provide for strains of isolated and purified bacteria which degrade 2-to 3-ringed low molecular weight PAHs such as naphthalene, phenanthrene, and fluoranthene along with PAH degradation intermediates.

It is yet a further aspect of at least one of the embodiments of the present invention to provide for a strain of isolated, purified bacteria which degrades 4-ring and higher molecular weight PAHs including pyrene and fluoranthene. Typically, the 4-ring and higher PAHs are much more persistent in the environment and resistant to degradation compared to low molecular weight PAHs. Accordingly, the ability to provide strains of bacteria which degrade 4-ring and higher high molecular weight PAHs is significant.

The PAH degradation intermediates may further function as metal chelators. This chelating activity or metal complexation may assist remediation in waste containing both PAHs and metals. Additionally, at least some of the bacterial strains identified herein have an ability to degrade several different types of PAHs (including 2-, 3-, and 4-ring PAHs) in addition to the ability to degrade phenanthrene.

It is yet another aspect of at least one of the embodiments of the present invention to provide strains of isolated and purified bacteria having surfactant properties useful in the removal of metals from contaminated soil and substrates. The isolated and purified strains of bacteria produce biosurfactant monomers. The biosurfactant monomers are produced in sufficient quantity that the monomers aggregate into three-dimensional structures including micelles. The biosurfactant micelles define polar head groups which bind with metal ions in the soil. The micelles, containing the metal ions, can be removed by aqueous suspensions or flushing, thereby lowering the metal ion content of the substrate. The resulting removed metals, contained within the biosurfactant micelles, are then more easily separated and concentrated for efficient disposal or storage.

It is yet another aspect of at least one of the present embodiments of the invention to provide for isolated and purified cultures of bacteria which produce biosurfactants under constitutive conditions, the isolated strains being further able to degrade PAHs during bioremediation conditions.

It is yet another aspect of at least one of the present embodiments of the invention to provide for isolated and purified strains of bacteria having the ability to degrade a broad range of different types of PAHs under bioremediation conditions.

It is yet another aspect at least one of the present embodiments of the invention to provide for isolated and purified strains of bacteria having an ability to bring about a general reduction of total petroleum hydrocarbons (TPH). In addition, the ability of certain of the isolates to produce a biosurfactant during bioremediation conditions increases the bioavailability of petroleum hydrocarbons to other microorganisms that may be present within the contaminated soil or other waste product.

It is yet another aspect of at least one of the present embodiments of the invention to provide for isolated and purified strains of bacteria having the ability to degrade PAHs along with an ability to produce a biosurfactant during bioremediation conditions.

It is yet another aspect of at least one of the present embodiments of the invention to allow for isolated and purified strands of bacteria having the ability to bring about a general reduction of lead availability which may be present within soil or lead containing waste streams. The biosurfactant produced by one or more of the isolates may be used in combination with inorganic phosphates to reduce the bioavailability of lead within a contaminated substrate such as soil.

Other aspects of at least one embodiment of this invention include a process of bioremediation of petroleum pollutants from a contaminated environment comprising the steps of providing a supply of a substrate contaminated with a petroleum pollutant; introducing into the supply of contaminated substrate at least one bacteria isolate which metabolizes constituents of the petroleum pollutant and which further produces a biosurfactant; and, providing adequate nutrients for a treatment time sufficient for the petroleum pollutant utilizing isolate to degrade the petroleum pollution to a target concentration of 100 ppm TPH or less.

These and other aspects of the invention are provided by biologically pure bacterial strains for bioremediation of petroleum products and PAHs comprising isolates selected from the isolates identified in Table 2.

These and other features, aspects, and advantages of the present invention will become better understood with reference to the following description and appended claims.

### BRIEF DESCRIPTION OF THE DRAWINGS

Figure 1 sets forth a graph showing the bioavailability of lead with respect to various additives and biological isolates with respect to highly contaminated soil (900mg Pb/Kg soil).
Figure 2 is a graph setting forth the changes in total organic matter within soil samples following a treatment protocol.

### DESCRIPTION OF PREFERRED EMBODIMENT

A fully and enabling disclosure of the present invention, including the best mode thereof, to one of ordinary skill in the art, is set forth more particularly in the remainder of the specification. Reference now will be made in detail to the embodiments of the invention, one or more examples of which are set forth below. Each example is provided by way of explanation of the invention, not limitation of the invention. In fact, it will be apparent to those skilled in the art that various modifications and variations can be made in the present invention without departing from the scope or spirit of the invention. For instance, features described as part of one embodiment can be used on another embodiment to yield a still further embodiment. Thus, it is intended that the present invention cover such modifications and variations as come within the scope of the appended claims and their equivalents. Other objects, features, and aspects of the present invention are disclosed in the following detailed description. It is to be understood by one of ordinary skill in the art that the present discussion is a description of exemplary embodiments only and is not intended as limiting the broader aspects of the present invention, which broader aspects are embodied in the exemplary constructions.

The present invention is directed to bacterial isolates obtained from a century-old Czechowice oil refinery in Poland. The aged sludge from the oil refinery is characterized by its acidic (pH 2) properties and contains high concentrations of PAHs along with heavy metals. Additionally, the sludge is characterized by the presence of spent catalysts, asphaltics, diatomaceous earth, silica gel, and coal fly ash, all containing high background levels of heavy metals (Pb/Cd/Zn) which have been previously deposited at the site. The collection site is from an area having approximately 120,000 tons of waste material deposited in unlined lagoons 3 meters deep covering an area of 3.8 hectares. A total of 45 bacteria, 68 fungi, and 7 yeast species were isolated from the sludge on an acidic minimum medium (pH 4) exposed to naphthalene vapor.

A subset of isolates was characterized by traditional taxonomic criteria, BIOLOG™_{,} and analysis of SSU rRNA genes. The bacterial groups included *Proteo* bacteria, *Ralstonia, Pseudomonas,* and *Alcaligenes* species. Further characterizations of the isolates may be seen in reference to the information provided in Tables 1 and 2. The BIOLOG™ characterization protocols using minimal nutritional factors along with various organic substrates of interest are described in reference to the publication Use of BIOLOG™ Technology for Hazardous Chemical Screening, Microbiological Techniques 18:329-347, 1993, and which is incorporated herein by reference.

A total of 45 bacteria, 68 fungi, and 7 yeast species were isolated using a naphthalene vapor acidic mineral salts basal growth medium. While not separately set forth, it is noted that many of the isolates have the ability to metabolize catechol and the bacterial isolates were characterized by the ability to degrade PAHs. Additionally, it is noted that the isolated and purified organisms having the ability to degrade PAHs also have the ability to degrade a variety of petroleum pollutants associated with measurements of total petroleum hydrocarbons.

As set forth below in Table 1, three bacterial species designated CZORL1B, BP20, and CZORL1Bsm, and which correspond to isolates 1 through 3 in Table 2, were observed to produce a surfactant when grown in a minimal medium containing naphthalene, phenanthrene, or fluoranthene. Nine additional strains identified in Table 1 were observed to degrade a range of PAHs indicating the isolates have a catalytic or enzymatic ability to degrade the contaminants although the additional isolates do not demonstrate an ability to produce a surfactant.

The above identified bacterial strains are grown and maintained on 1 percent peptone, trypticase, yeast extract, glucose (PTYG) plates. The bacteria were grown aerobically at 30° C and maintained on a minimal medium at 4° C or long-term storage in a frozen medium maintained at -70°C or in liquid nitrogen (-196° C).

The identification of the bacteria was made using rDNA or Fatty Acid Methyl Esters (FAME) identification protocols as set forth in the publication Bacterial Evolution, Microbial Reviews 51:221-271 by C. R. Woese (1987) and which is incorporated herein by reference. Deposits of isolates 1 through 12 as identified in Table 2 were deposited with the American Type Culture Collection (ATCC), Rockville, Maryland, on October 9, 2003, and have the indicated ATCC designation numbers. The deposit forms accompanying each of the isolate deposits as submitted to the ATCC are incorporated herein by reference.

**Table 1**

| Dihydroxylating dioxygenase and 2,3 catechol dioxygenase activity and PAH degradative range of isolates. | | | | | | | | | |
|---|---|---|---|---|---|---|---|---|---|
| Assayed for the following characteristlcs:^{a} | | | | | | | | | |
| Isolate | indigo | meta fission | NAP | PHE | ANT | FLE | ACE | FLA | PYR |
| | | | | | | | | | |

| Isolated on naphthalene vapor^{b} | | | | | | | | | |
|---|---|---|---|---|---|---|---|---|---|
| BAA-1 (PB19) | indigo | | | | | | | | |
| BAA (PB16) | indigo | | | | | | | | |
| BP19A(PB17) | indigo | | | | | | | | |
| BAB (PB14) | indigo | | | | | | | | |
| CZORL1B (KN-1)^{c} | | meta fission | | | | | | | |
| BP20 CZORL1B (KN-2)^{c} | | meta fission | | | | | | | |
| CZORL1Bsm (KN-3)^{c} | | meta fission | | | | | | | |
| PB15 | indigo | | NAP (was designated BP20) | | | | | | |
| | | | | | | | | | |

| Isolated with phenanthrene overspray^{d} | | | | | | | | | |
|---|---|---|---|---|---|---|---|---|---|
| BPA | indigo | | | PHE | ANT | FLE | | | |
| BPB | indigo | | | PHE | ANT | FLE | | | |
| BPC | indigo | | | PHE | ANT | FLE | | | |
| BPD | indigo | | | PHE | ANT | FLE | | | |
| BPE | indigo | | | PHE | ANT | FLE | | | |
| BPF | indigo | meta fission | NAP | PHE | ANT | FLE | ACE | FLA | PYR |
| BPG | indigo | meta fission | | | | | | | |
| BPH | indigo | meta fission | | PHE | ANT | FLE | | FLA | PYR |
| BPI | indigo | | | PHE | ANT | FLE | | | |
| BPJ | indigo | | | | ANT | FLE | | | |
| BPK | | | | PHE | ANT | FLE | | | |
| BPL | indigo | | | PHE | | FLE | | | |
| BPM | indigo | | | PHE | ANT | FLE | | | PYR |
| BPN | indigo | | | PHE | ANT | FLE | | FLA | |
| BPO | indigo | | | PHE | ANT | FLE | | | |
| BPP | indigo | | | PHE | | | | | |
| BPQ | indigo | | | PHE | ANT | FLE | | | |
| BPR | indigo | meta fission | | PHE | | | | | |

| | | | | | | | | | |
|---|---|---|---|---|---|---|---|---|---|
| a Abbreviations; indigo, production of indigo from indole; meta fission, 2,3 catechol dioxygenase activity; NAP, naphthalene; PHE, phenanthrene; ANT, anthracene; FLE, fluorene; ACE, acenanphthene; FLA, fluoranthene; PYR, pyrene. b Bacteria isolated on agar plates exposed to naphthalene. c Bacteria produced biosurfactant d Bacteria isolated on agar plates exposed to phenanthrene crystals. | | | | | | | | | |

**Table 2.**

| **Isolate identification.** | | | |
|---|---|---|---|
| **Isolate** | | **Identification** | **ATCC Accession Number** |
| | | | |
| 1) | CZOR-L1B (KN-1) | *ALCALIGENES-PIECHAUDII SRS* | PTA-5580 |
| | | | |
| 2) | BP-20 (KN-2) | *RALSTONIA PICKETTII SRS.* | PTA-5579 |
| 3). | CZOR-L1Bsm(KN-3) | *PSEUDOMONAS-PUTIDA* BIOTYPE B *SRS* | PTA-5581 |
| 4) | BPB | *FLEXIBACTER CF. SANCTI SRS* | PTA-5570 |
| 5) | BPC | *PSEUDOMONAS FREDRIKSBERGENSIS SRS* | PTA-5571 |
| 6) | BPE | *STAPHYLOCOCCUS WARNERI. LMG 19417 SRS* | PTA-5572 |
| 7) | BPF | *SPHINGOMONAS SRS* | PTA-5573 |
| 8) | BPH | *SPHINGOMONAS SP. S37 SRS* | PTA-5574 |
| 9) | BPI | *PHYLOBACTERIUM SRS* | PTA-5575 |
| | | (α PROTEOBACTERIUM TA-A1) | |
| 10) | BPJ | *SERRATIA FICARIA SRS* | PTA-5576 |
| | | (α PROTEOBACTERIUM TA12-21) | |
| 11) | BPK | *AGROBACTERIUM TUMEFACIENS SRS* | PTA-5577 |
| 12) | BPL | *RHIZOBIUM SP. SDW045 SRS* | PTA-5578 |

The above identified bacteria isolates have been established as distinct species. Each of the identified isolates has PAH-degrading properties and have demonstrated an ability to reduce TPH in soil as well. In addition, certain isolates have the ability to produce a biosurfactant. Each isolate is believed novel, based upon the rDNA characterization and variations noted in Tables 1 and 2 with respect to physiological growth characteristics.

The isolates 1-3, ATCC PTA-5580(Alcaligenes *piechaudii SRS*)*;* ATCC PTA-5579, (*Ralstonia pickettii SRS*)*;* and ATCC PTA-5581 (*Psuedomonas putida* Biotype B *SRS*) identified above, all demonstrate the ability to produce a biosurfactant, the formation of which was noted during culturing conditions. The biosurfactant exudate was evaluated for each isolate and determined to have a surface tension altering property consistent with a surfactant. Isolates 4-12 all demonstrate the ability to biodegrade a variety of PAHs (Table 1). As set forth in Example 1 below, the use of a consortium of the twelve isolates identified in Table 2 to remediate petroleum hydrocarbons contained in soil in a bioreactor remediation study results in visible quantities of biosurfactants being produced under the bioremediation conditions.

The ability of certain of the isolates to produce bioreactants is believed to enhance remediation through several different mechanisms. The production of the biosurfactant increases the biological availability of PAHs and other hydrophobic petroleum compounds. The increased biological availability includes the ability of the produced surfactant to solubilize and make available to the isolate the PAHs and other petroleum compounds. As such, the isolates' ability to produce surfactants increases the efficiency of the isolates to degrade and metabolize PAHs.

As noted in Example 1, the consortium of isolates used results in visible quantities of surfactants being produced within the soil. Biosurfactants are generally known to have a chemistry consisting of a polar head and a non-polar tail. In aqueous solutions, biosurfactants serve to reduce liquid surface tension and to facilitate the formation of an emulsion between liquids of different polarities. This ability facilitates the biosurfactants' usefulness in that hydrophobic, non-polar tail regions of the biosurfactants and biosurfactant micelles may trap oils and other petroleum compounds. The trapped oils and petroleum compounds have greater bioavailability to bacteria for biodegradation. Additionally, micelles containing trapped oils and petroleum compounds may be periodically removed or flushed from the system, thereby providing an ability to further isolate and separate petroleum compounds from the soil substrate.

Additionally, micelles formed by the biosurfactants promote the removal of metals from the soil. The hydrophilic polar head groups of micelles will bind metal and metal ions present within the soil. Once bound, the soluble nature of the micelles allows the micelles and bound metals to be collected. Once collected, the now concentrated volume of micelles and contained metals can be further treated to separate the metals from the biosurfactant. In addition, it has been found that selected isolates which produce biosurfactants can be used to reduce the bioavailability of certain metals such as lead present within the soil. For certain remediations, the surfactants are not used to remove a contaminant, but rather provide a method of long term stabilization of a contaminated site. Stabilization includes reducing the bioavailability of lead along with preventing the migration of lead in surface runoff to adjacent, uncontaminated sites and groundwater.

### Example 1

A mobile bioreactor was constructed and was supplied with a four ton volume of soil contaminated with low level cesium-137 and 26,000 ppm petroleum hydrocarbons. The contaminated soil was weathered material obtained from the Savannah River Site (Aiken, SC). The source and make up of the petroleum products is unknown but believed to be a mixture of used motor oil and diesel fuel.

The soil was amended with a 7% bulking agent of aged compost. For each isolate, a three liter culture in log growth phase was added and distributed within the four tons of mixed waste soil. The bioreactor is equipped with a raised, secondary, perforated floor having bottom feed aeration lines which provide a continuous supply of ambient air to the bioreactor. Additionally, periodic nutrient supplements of nitrogen, potassium, and phosphorus fertilizers (10-10-10) were applied to enhance the biological activity within the bioreactor. Influent and effluent water couplings were attached. Air compressors, vacuum pumps, and a liquid pump were used to control and regulate the air and liquid flows through the bioreactor and control moisture content In the bioreactor.

The presence of low level cesium-137 limited the number and types of sampling techniques used to monitor the bioreactor and required the use of HEPA filtering with the air effluent couplings. Periodic CO₂ measurements indicated that, in a five-month interval, 121 pounds of petroleum products were degraded. Based upon the CO₂ measurements, it is conservatively estimated that the bioremediation process removed 16,000 mg/kg of petroleum contaminants from the soil during the five-month evaluation interval. It was demonstrated that the 4 ton volume of contaminated soil had the TPH reduced to 45 ppm after 20 months of treatment. The ability of the isolates to degrade petroleum and other hydrocarbon products while producing biosurfactants offers enormous advantages in terms of efficiency and versatility of treatment protocols. For instance, it is believed that for soils contaminated solely with petroleum and petroleum by-products, the present isolates may, either individually or as a consortium, be used with conventional bioremediation techniques to improve the efficiency of degradation. As described above, the action of the biosurfactants creates a greater zone of petroleum solubility for each individual bacterium. As a result, a greater availability of petroleum products occurs. Further, to the extent the isolates form aggregate colonies, biofilms, or biosheets within portions of the soil, the surfactants are believed to substantially increase the bioavailability of the petroleum substrates for the bacterial aggregates. At the same time, the surfactants also increase the solubilization of heavy metals that may be present and provide an ability to reduce the heavy metal concentration by removal of the produced surfactants.

As seen in Example 1, the consortium of isolates provides for a bioremediation process which can achieve significant reductions in petroleum from contaminated soil. This property is particularly useful with respect to formulating disposal strategies for mixed waste in which petroleum contaminated soil and low- level radioactive material are present together. Currently, soil contaminated with low-level radioactive waste and having additional petroleum contaminants must be below regulatory limits of 1 ppm for BTEX (benzene, toluene, ethylbenzene, xylene, and 100 ppm TPH (Total Petroleum Hydrocarbons) before the soil can be classified and disposed of as a low-level radioactive waste. The cost of disposing of soil which meets the definition of a low-level radioactive waste is approximately U.S. $262 per cubic meter per year. In contrast, soil containing both low-level radioactive material and petroleum contamination in excess of the regulatory limits must be stored as a mixed waste product. The cost of storage of mixed waste soil is approximately U.S. $10,165 per cubic meter of soil per year based on yearly costs alone. The ability to treat mixed waste soils and thereby remove substantial levels of petroleum contaminants is of critical importance. Removing sufficient petroleum contaminants from a mixed waste soil allows the waste to be disposed of as a low-level radioactive waste. The resulting cost is 38 times lower than the storage cost of a mixed waste.

The use of the present inoculants is further advantageous in that, unlike some prior art techniques, the volume of amendments to the soil is kept at a minimum. Keeping the volume of soil amendments to a minimum reduces the eventual disposal costs, particularly for soil containing low-level radiation.

The present isolates are also believed useful for *in situ* remediation projects. The consortium of isolates may be supplied to contaminated soil using any number of conventional techniques. As needed, nutritional supplements along with the supply of oxygen in either a physical or chemical form facilitates the bioremediation activity. Given the isolates' ability to degrade PAHs as well as the desired ability to degrade petroleum hydrocarbons generally, *in situ* remediation using the isolates is advantageous. Additionally, the ability of certain of the isolates to produce a surfactant (biosurfactant) during soil growth conditions makes the use of certain isolates more beneficial. As noted, the biosurfactant enhances the ability to physically entrap petroleum products and heavy metals as well as providing for increased solubilization and access of petroleum hydrocarbons to both the bacterial isolates as well as native microorganisms present within the soil environment.

The use of biosurfactants can bring about useful results in a variety of contaminated substrate conditions. For instance, it is known in the art that biosurfactants have useful metal-complexing properties that can both reduce metal toxicity while allowing enhanced biodegradation of other waste in sites contaminated with both organic and metal pollutants. One such publication, entitled "A Rhamnolipid Biosurfactant Reduces Cadmium Toxicity during Naphthalene Biodegradation", by Sandrin et al, Applied and Environmental Microbiology, Oct. 2000, pp 4585-4588, and which is incorporated herein by reference, describes the ability of biosurfactants to reduce metal toxicity in co-contaminated systems having both organic and metal pollutants.

An additional publication, "Microbial Surfactants and their use in Field Studies of Soil Remediation", by N. Christofi and I.B. Ivshina, published in the Journal of Applied Microbiology, 2000, 93, pp. 915-929, and which is incorporated herein by reference, provides a useful overview of the role of surfactants in soil remediation applications. Biosurfactants can, under appropriate conditions, provide useful benefits with respect to organic solubilization, organic degradation, metal solubilization, and metal isolation.

Certain isolates disclosed herein have demonstrated an ability to reduce the bioavailability of lead within a contaminated soil. Lead contaminants in soil may occur as a result of numerous activities including mining, presence of lead-based paint, ordnance from firing ranges, contamination from gasoline and petroleum additives, lead battery recycling centers, and residue from lead-based explosives. Lead exposure is associated with numerous disorders human nervous and reproductive systems. Lead usually enters the body through inhalation or ingestion of lead-containing dust.

It is known that lead containing soils can be treated with the use of soil amendments to reduce the bioavailability of lead. Some such amendments include apatites, HR, and other calcium phosphate containing materials. Apatites are known to bind with lead and other metals within soil and thereby reduce the tendency of lead to migrate. The binding interaction also reduces the bioavailability of lead. Selected surfactants may also be used to reduce the bioavailability of lead as demonstrated by isolate 3.

An evaluation of isolate 1 (*Alcaligenes piechaudii*) and isolate 3 (*Pseudomonas putida*) was conducted to see if beneficial reductions in lead bioavailability could be obtained by incorporating the biosurfactant producing isolates along with other soil amendments in lead-containing soil. The lead contaminated soil was obtained from a small arms firing range where the lead bullets had accumulated in the soil over a number of years. 50 gm samples of contaminated soil were mixed with 3 different calcium phosphate amendments. The calcium phosphate amendments included a naturally occurring rock calcium phosphate from North Carolina (USA) hereinafter designated as NCA, a naturally occurring rock calcium phosphate from Florida (USA) hereinafter designated FA, and a biological apatite obtaining from ground fish bones, hereinafter designated as BA. 10% and 5% by weight additions of the NCA, SA, and BA were made to the respective samples of the lead contaminated soils. Additionally, certain of the amended soil samples were further treated by the introduction of 5 ml samples of the isolate number 1, *Alcaligenes piechaudii* in a 1 % PTYG nutrient broth. Density of the isolate was 3.11 E + 08 cells per ml. Additional samples were also treated with isolate number 3 *Pseudomonas putida* using a similar 5 ml addition and a density of 3.43 E + 08 cells per ml containing samples for the microbial amendment included a 1% PTYG sterile broth. The contaminated soil samples and various amendments and cultures were mixed together in sealed containers which were connected by tubing to a Micro-Oxymax Respirometer (Columbus Instruments, Columbus, OH) for determination of metabolic rates includes oxygen consumption and CO₂ evolution. Microbial density was obtained through conventional plating techniques for determining colony and sample density.

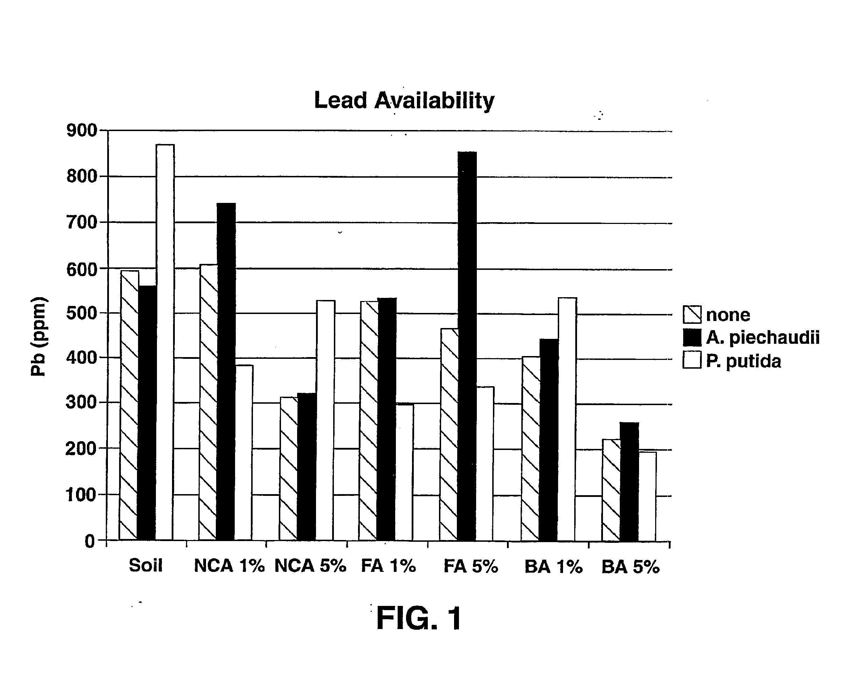

As seen in the results set forth in Figure 1, the addition of isolate 3 achieves a significant decrease in lead bioavailability as measured through a toxicity characteristic leaching procedure (TCLP) using protocols identified in the U.S. Environmental Protection Agency Method 6010. The microbial amendments brought about significant reductions in lead bioavailability when used with the 5% soil amendments of NCA or BA. Given the low cost of a BA product and uniformity of the biological apatite, it is believed that use of the biological apatite in combination with the isolate 3, or combinations thereof, offers an excellent reduction for the bioavailability of lead within soil.

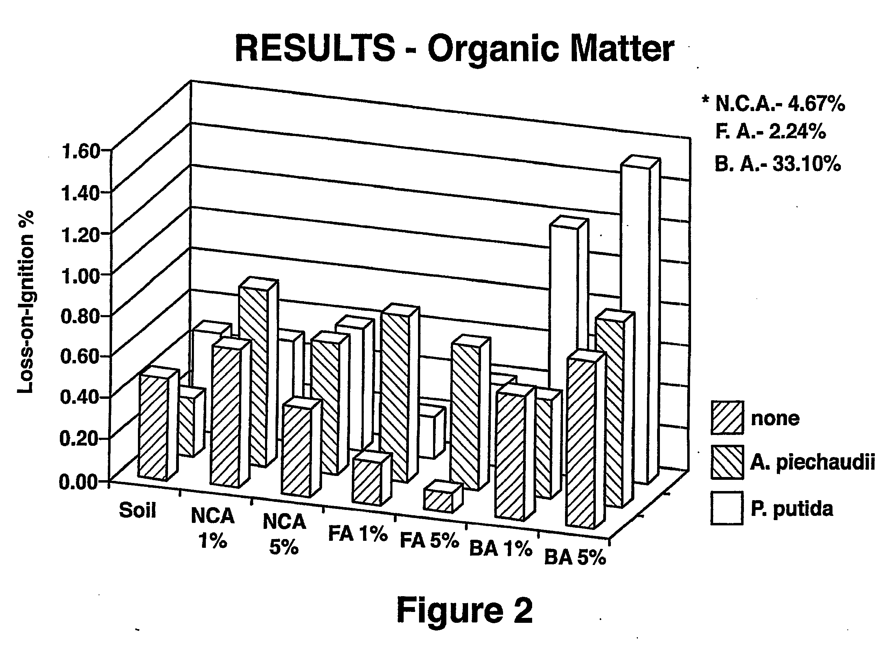

The combination of the biological apatite with the naturally producing biosurfactants from isolate 3 bacteria brings about other noted improvements to the contaminated soil samples. It is noted that an increase in metabolic rates as measured by oxygen consumption and carbon dioxide production increased along with the overall microbial density. As seen in reference to Figure 2, the total organic matter is significantly increased following a 114 hour incubation period for samples using the *P. putida* isolate in combination with the biological apatite at both 1 % and 5% incorporation levels. The increase in total organic matter reflects the increase in total microbial population attributable to the increase nutrient supply of the biosurfactants. Increased availability of micronutrients results in enhanced biological activity resulting in an increase in organic matter. While the most significant results can be seen with the biological apatites in combination with the *P. putida* isolate strain, various combinations of other apatites suggests that one or more isolates may be used to increase total microbial activity.

While not separately reported, it is noted that the increases in organic matter as noted in Figure 2 correlated with significant increases in soil pH, O₂ consumption, CO₂ production, as well as the reduced lead bioavailability as noted in Figure 1.

It is believed that the use of the isolates described above bring about a decrease in bioavailability of lead due at least in part to the surfactant producing properties of the isolates. The biosurfactants are believed to accentuate the inherent tendencies of the apatite with respect to increasing metabolic rates, nutrient availability, and favorable increases in pH and lowering of lead bioavailability. As previously described, the biosurfactants bring about favorable soil conditions that improve nutrient conditions and availability of micronutrients for micro-organisms. The increase in biological activity and associated biomass within the soil is believed to further decrease the lead bioavailability. The *P*. *putida* activity of lowering soil lead bioavailability is synergistic with BA at the 5% concentration. The exponential increase in respiration rate, microbial density and increase in biomass is clear evidence that the activity is related to specific biological activity.

As noted in Figure 1, different surfactants have different results when applied to any particular apatite used as the inorganic additive. One could, through routine experimentation, evaluate the various isolates to determine the combination offering the desired improvements in lead bioavailability with respect to the given soil conditions, selected apatites and/or other additives used in the remediation procedure. The most useful combinations of additives and isolates can be selected to bring about the desired reduction in lead bioavailability.

As described previously in this application, the isolates are useful for reduction of PAHs as well as facilitating the removal and/or sequestration of mineral ions from contaminated soils and substrates. Accordingly, for contaminated soils and waste streams having a variety of contaminants including PAHs, metal ions, and other metals such as lead, use of one or more of the present isolates, including isolates producing a biosurfactant, can effectively treat multiple contaminants within a given site.

While the above examples were given in the context of remediation of a highly contaminated soil (900mg Pb/Kg soil), it is believed that the utility of individual isolates or combinations of isolates is not limited to soil remediation efforts *per se.* For instance, it is believed that the isolates producing biosurfactants can be used as part of a waste stream treatment process of a mine or industrial facility where heavy metals, metal ions, lead, copper, cadmium, zinc, other metals, and other contaminants are generated. Many sites (i.e., landfills and brownfields) have lead contamination in the low ppm concentration (1-10mg Pb/Kg soil) that is still of health concern due to the potential toxicological impact and future land use. The use of inoculants which are incorporated into an initital waste stream and which include one or more of the above isolates can bring about a reduced volume of contaminants within the waste stream and introduce a microbial flora population which can persist as part of a longer term treatment protocol for generated and/or stored waste. For instance, mine tailings can be initially treated to reduce certain contaminants prior to being deposited onto a disposal area. Afterwards, the presence of the isolates within the mine tailings can be used to advantage by further treatment designed to promote the useful biological activity so as to bring about a continuing reduction and/or stabilization of the contaminants present in the waste stream.

It is believed that similar results can be used in a variety of industrial waste streams where the isolates can be used as part of a biological treatment process to initially reduce the level of contaminants and to provide a microbial population which can be used as part of a longer term treatment for the stored or deposited waste streams.

Although preferred embodiments of the invention have been described using specific terms, devices, and methods, such description is for illustrative purposes only. The words used are words of description rather than of limitation. It is to be understood that changes and variations may be made by those of ordinary skill in the art without departing from the spirit or the scope of the present invention, which is set forth in the following claims. In addition, it should be understood that aspects of the various embodiments may be interchanged, both in whole and in part. Therefore, the spirit and scope of the appended claims should not be limited to the description of the preferred versions contained therein.

Other aspects of the invention include the following:
Aspect 1. A biologically pure bacterial strain for bioremediation of petroleum hydrocarbons comprising an isolate selected from the group consisting of ATCC accession numbers PTA-5570, PTA-5571, PTA-5572, PTA-5573, PTA-5574, PTA-5575, PTA-5576, PTA-5577, PTA-5578, PTA-5579, PTA-5580, PTA-5581 and combinations thereof.
Aspect 2. A process of bioremediation of petroleum pollutants from a soil environment comprising the steps of:
   providing a supply of a contaminated soil having a petroleum pollutant;
   introducing into said supply of contaminated soil at least one bacteria isolate which metabolizes constituents of the petroleum pollutant and which further produces a biosurfactant; and,
   providing adequate nutrients for a treatment time sufficient for the pollutant utilizing isolate to degrade the petroleum pollution.
Aspect 3. The process according to Aspect 2 wherein said concentration of petroleum pollutant within said soil is less than about 100 ppm total petroleum hydrocarbons after treatment with the at least one bacterial isolate.
Aspect 4. A process of treating a mixed waste of soil contaminated with a heavy metal pollutant and a petroleum pollutant comprising the steps of:
   providing a supply of a contaminated soil containing a heavy metal pollutant and a petroleum pollutant;
   introducing into said supply of contaminated soil at least one bacterial isolate which metabolizes a constituent of the petroleum pollutant and which further produces a biosurfactant; and,
   periodically removing a portion of the produced biosurfactant from said supply of soil, said biosurfactant containing therein either a portion of said heavy metals or said petroleum pollutants; and,
   repeating said step of removing a portion of said surfactant until said heavy metal concentration is reduced to a target value.
Aspect 5. The process according to Aspect 2 wherein said supply of a contaminated soil further contains a low-level radioactive waste.
Aspect 6. The process according to Aspect 2 wherein said step of introducing an isolate further comprises adding an isolate selected from the group consisting of ATCC accession numbers PTA-5579, PTA-5580, and PTA-5581 and combinations thereof.
Aspect 7. The process according to Aspect 4 wherein said step of introducing at least one bacterium isolate further comprises adding an isolate selected from the group consisting of ATCC accession numbers PTA-5579, PTA-5580, and PTA-5581 and combinations thereof.
Aspect 8. A process of treating a soil containing low-level radioactive material and petroleum hydrocarbons comprising the steps of:
   providing a supply of contaminated soil containing a radioactive waste and a petroleum hydrocarbon;
   introducing into said supply of contaminated soil at least one bacterial isolate which metabolizes a constituent of the petroleum hydrocarbon and is selected from the group consisting of ATCC accession numbers PTA-5570, PTA-5571, PTA-5572, PTA-5573, PTA-5574, PTA-5575, PTA-5576, PTA-5577, PTA-5578, PTA-5579, PTA-5580, PTA-5581, and combinations thereof which metabolize a constituent of the petroleum hydrocarbons; and,
   treating said supply of contaminated soil until a treated concentration of said petroleum pollutant is less than about 100 ppm total petroleum hydrocarbons.
Aspect 9. The process according to Aspect 8 wherein said at least one bacterial isolate further comprises an isolate selected from the group consisting of ATCC accession numbers PTA-5579, PTA-5580, and PTA-5581, and combinations thereof.
Aspect 10. The process according to Aspect 2 wherein said petroleum pollutant includes polyaromatic hydrocarbons.
Aspect 11. The process according to Aspect 10 wherein said polyaromatic hydrocarbons further includes polyaromatic hydrocarbons selected from the group consisting of 2-ringed, 3-ringed, and 4-ringed polyaromatic hydrocarbons and combinations thereof.
Aspect 12. The process according to Aspect 8 wherein said at least one bacterial isolate additionally produces a surfactant during bioremediation conditions.
Aspect 13. The process according to Aspect 12 comprising the additional step of periodically removing a portion of the produced surfactant from said supply of contaminated soil.
Aspect 14. The process according to Aspect 4 wherein said petroleum pollutant further includes polyaromatic hydrocarbons.
Aspect 15. The process according to Aspect 14 wherein said polyaromatic hydrocarbons further includes polyaromatic hydrocarbons selected from the group consisting of 2-ringed, 3-ringed, and 4-ringed polyaromatic hydrocarbons and combinations thereof.
Aspect 16. A process of treating lead contaminated soil so as to reduce the bioavailability of lead comprising:
   providing a supply of the lead contaminated soil;
   providing a soil amendment in the form of an about 1 % to about 5% or greater of a biological apatite;
   further adding to said contaminated soil an innoculant selected from the group consisting of ATTC accession numbers PTA-5580, PTA-5581, and combinations thereof;
   wherein said contaminated soil hereafter has a lower bioavailability of lead.

## Claims

1. A combination of biologically pure bacterial strains for bioremediation of petroleum hydrocarbons comprising isolates of ATCC accession numbers PTA-5570, PTA-5571, PTA-5572, PTA-5573, PTA-5574, PTA-5575, PTA-5576, PTA-5577, PTA-5578, PTA-5579, PTA-5580, and PTA-5581.

2. A process of bioremediation of petroleum pollutants from a soil environment comprising the steps of:
providing a supply of a contaminated soil having a petroleum pollutant;
introducing into said supply of contaminated soil the combination of claim 1 which metabolizes constituents of the petroleum pollutant and which further produces a biosurfactant; and,
providing adequate nutrients for a treatment time sufficient for the petroleum pollutant utilizing said combination to degrade the petroleum pollution.

3. The process according to claim 2 wherein said concentration of petroleum pollutant within said soil is less than about 100 ppm total petroleum hydrocarbons after treatment with the combination.

4. A process of treating a mixed waste of soil contaminated with a heavy metal pollutant and a petroleum pollutant comprising the steps of:
providing a supply of a contaminated soil containing a heavy metal pollutant and a petroleum pollutant;
introducing into said supply of contaminated soil at least one bacterial isolate which metabolizes a constituent of the petroleum pollutant and which further produces a biosurfactant; and,
periodically removing a portion of the produced biosurfactant from said supply of soil, said biosurfactant containing therein either a portion of said heavy metals or said petroleum pollutants; and,
repeating said step of removing a portion of said surfactant until said heavy metal concentration is reduced to a target value.

5. The process according to claim 2 wherein said supply of a contaminated soil further contains a low-level radioactive waste.

6. A process of treating a soil containing low-level radioactive material and petroleum hydrocarbons comprising the steps of:
providing a supply of contaminated soil containing a radioactive waste and a petroleum hydrocarbon;
introducing into said supply of contaminated soil the combination of claim 1 which metabolize a constituent of the petroleum hydrocarbons; and
treating said supply of contaminated soil until a treated concentration of said petroleum pollutant is less than about 100 ppm total petroleum hydrocarbons.

7. The process according to claim 2 wherein said petroleum pollutant includes polyaromatic hydrocarbons.

8. The process according to claim 7 wherein said polyaromatic hydrocarbons further includes polyaromatic hydrocarbons selected from the group consisting of 2-ringed, 3-ringed, and 4-ringed polyaromatic hydrocarbons and combinations thereof.

9. The process according to claim 6 wherein said at least one bacterial isolate of said combination additionally produces a surfactant during bioremediation conditions.

10. The process according to claim 9 comprising the additional step of periodically removing a portion of the produced surfactant from said supply of contaminated soil.

11. The process according to claim 4 wherein said petroleum pollutant further includes polyaromatic hydrocarbons.

12. The process according to claim 11 wherein said polyaromatic hydrocarbons further includes polyaromatic hydrocarbons selected from the group consisting of 2-ringed, 3-ringed, and 4-ringed polyaromatic hydrocarbons and combinations thereof.
